# EUROPÄISCHE PATENTSCHRIFT

(11) **EP 3 881 750 B1**
(45) Veröffentlichungstag und Bekanntmachung des Hinweises auf die Patenterteilung: **22.05.2024**
(21) Anmeldenummer: 21162119.8
(22) Anmeldetag: 11.03.2021
(51) Int. Cl.: A61B 1/00

(54) **STECKVERBINDUNG FÜR EIN VIDEOENDOSKOP, VIDEOENDOSKOP UND VERFAHREN ZUR HERSTELLUNG EINER STECKVERBINDUNG**
CONNECTION FOR A VIDEO ENDOSCOPE, VIDEO ENDOSCOPE AND METHOD FOR PRODUCING A CONNECTION
CONNECTEUR ENFICHABLE POUR UN VIDÉO-ENDOSCOPE, VIDÉO-ENDOSCOPE ET PROCÉDÉ DE FABRICATION D'UN CONNECTEUR ENFICHABLE

(30) Priorität: 18.03.2020 DE 102020107492
(43) Veröffentlichungstag der Anmeldung: 22.09.2021
(73) Patentinhaber: OLYMPUS Winter & Ibe GmbH, 22045 Hamburg (DE)
(72) Erfinder: THÜMEN, Alrun, 22043 Hamburg (DE); JUNGBAUER, Sebastian, 22767 Hamburg (DE)
(74) Vertreter: Seemann & Partner Patentanwälte mbB

(56) Entgegenhaltungen:
- EP-A1- 1 839 559
- EP-A1- 1 859 726
- WO-A1-2016/146393
- US-A1- 2015 335 230

## Beschreibung

Die Erfindung betrifft eine Steckverbindung für ein Videoendoskop zur Herstellung einer Signalverbindung zwischen einer distalen Videoeinheit und einer proximalen Kabeleinheit des Videoendoskops, ein entsprechendes Videoendoskop sowie ein Verfahren zur Herstellung einer entsprechenden Steckverbindung.

In Videoendoskopen gibt es eine Steckverbindung zwischen der distalen Videoeinheit, auch R-Unit genannt, die üblicherweise etwa den Endoskopschaft und den Bildsensor sowie dessen Ausleseelektronik beinhaltet, und der proximalen Kabeleinheit, auch Cable-Unit genannt, die üblicherweise den Handgriff umfasst. Diese Steckverbindung verbindet die Videoleitungen der Videoeinheit mit denen des Kabels. Auf der Seite der Videoeinheit gibt es einen 30-Pin-Stecker mit Glasverguss, einem O-Ring und einem Spiralfederring zur Abdichtung und Sicherung. Diese Feder und der O-Ring funktionieren nicht immer und werden verhältnismäßig schnell beschädigt.

Der Konnektor auf der Seite der Kabeleinheit wird aus mehreren Teilen zusammengebaut. Die Kabel werden manuell in einem mehrstufigen Prozess abisoliert und der äußere Schirm in eine Ferrule, d.h. einen Stopfen mit Durchgängen für die Kabel, eingelötet. Danach werden die einzelnen Litzen in Lötkelche des Konnektors eingelötet, teilweise wird dafür noch zusätzlich ein Lötadapter für die dickeren Litzen benötigt. Viele der Einzelleitungen sind Koaxial-Kabel. Deren Schirme werden verdrillt, in Gruppen zusammengefasst und auch in Lötkelche gelötet. Anschließend wird der Konnektor in das Gehäuse eingepresst. Danach wird der Innenraum mit einem Dickschichtlack bis zum Gewinde vergossen und anschließend der Stecker mit dem Mount. Als letztes wird die Ferrule mit dem Deckel verlötet. Die Steckverbindung wird dann mit Schrauben und Federringen gesichert.

Diese manuelle Montage des Steckers ist aufwändig und fehleranfällig. Der Stecker ist nicht gut zu stecken, da die Vorausrichtung der Stecker zueinander nur optisch und nicht taktil möglich ist. Dadurch ist nicht gewährleistet, dass die Durchkontaktierungen (Pins) direkt in den Sockel des Gegenstücks greifen. Weiterhin sind zwei Schrauben mit Federscheiben verspannt anzuziehen, was lange dauert und fehleranfällig ist. Das Anlöten der Kabel an die Lötkelche ist schwierig da die Kabel unterschiedliche Leitungsdurchmesser haben. Die Schirme der Koaxialkabel müssen teilweise zu Gruppen verdrillt werden. Diese Gruppen können unterschiedlich dick werden und müssen auch an die Kelche gelötet werden. Da der Bauraum sehr klein ist, ist dieses Anlöten schwierig und fehleranfällig, so dass es zu häufigen Nacharbeiten kommt. Ferner wird die äußere Kabelschirmung an die Ferrule angelötet. Da diese rund ist, kann sie sich während der Montage mitdrehen. Dadurch können sich die schon angelöteten Kabel im Inneren verdrehen und dadurch verkürzen. Die Lötungen werden dadurch belastet und ggf. vorgeschädigt, was zu frühen Ausfällen in der Benutzung führen kann.

Die unsichere Fixierung, unklare Wasserdichtigkeit und große Bauform des Steckers sind weitere Punkte, die die Handhabung erschweren. Die Feder und der O-Ring funktionieren nicht immer bzw. werden schnell beschädigt. Der Verguss reicht nur bis zum Gewinde, wodurch die Kabel im hinteren Teil des Konnektors ungeschützt sind. Die Befestigungsösen bzw. -augen des Steckers sind sehr ausladend und lassen kein schmaleres Handgriffdesign zu.

Auch die elektrische Signaltransmission ist nicht ideal. Um die inneren koaxialen Leitungen an die Lötkelche des Steckers anbringen zu können, müssen Schirmgruppen erstellt werden. Weiterhin ist es notwendig die Innenleiter in langen Bögen zu führen. Diese Ausführung stellt einen großen Sprung in der Transferimpedanz des Steckers dar und ist für schnelle Digitalsignale ungünstig.

US 2015/0335230 A1 offenbart eine entsprechende Steckverbindung für ein Videoendoskop zur Herstellung einer Signalverbindung zwischen einer distalen Videoeinheit und einer proximalen Kabeleinheit. Darin umfasst ein fotoelektrisches Verbundmodul ein erstes Verbindungselement mit einem röhrenförmigen Außenrahmen und einer Vielzahl von Kontakten, die sich im Inneren des Außenrahmens befinden; eine erste Leiterplatte, auf der ein fotoelektrisches Wandlerelement angebracht ist, das so konfiguriert ist, dass es ein elektrisches Signal in ein optisches Signal umwandelt und als Relais zwischen den Kontakten und dem fotoelektrischen Wandlerelement wirkt; und eine zweite Leiterplatte, die so konfiguriert ist, dass sie als Relais zwischen den Kontakten und einem elektrischen Signalkabel wirkt. Die erste Leiterplatte und die zweite Leiterplatte sind dreidimensional angeordnet.

Demgegenüber liegt der vorliegenden Erfindung die Aufgabe zugrunde, eine Steckverbindung, ein Videoendoskop und ein Verfahren zur Herstellung einer solchen Steckverbindung bereitzustellen, das die genannten Nachteile überwindet.

Diese Aufgabe wird durch eine Steckverbindung für ein Videoendoskop zur Herstellung einer Signalverbindung zwischen einer distalen Videoeinheit und einer proximalen Kabeleinheit des Videoendoskops gelöst, umfassend einen Stecker und einen komplementären Konnektor, der zur Herstellung und Unterbrechung der Signalverbindung in den Stecker einsteckbar ist, wobei der Stecker als Glasvergussteil geformt und an einem proximalen Ende der distalen Videoeinheit angeordnet ist und der Konnektor an einem distalen Ende der proximalen Kabeleinheit angeordnet ist, wobei der Stecker an seiner Außenseite mit einem Gehäuse der distalen Videoeinheit verbunden ist, eine Mehrzahl von elektrischen Durchkontaktierungen aufweist, auf einer distalen Seite mit einem Flexboard in der distalen Videoeinheit elektrisch leitend verbunden ist und auf seiner proximalen Seite eine als Aussparung ausgebildete Konnektoraufnahme für den Konnektor aufweist, wobei der Konnektor in einem Konnektorgehäuse eingefasst ist, welches an seiner Außenseite passend zu der Konnektoraufnahme des Steckers geformt ist, um formschlüssig und/oder kraftschlüssig in der Konnektoraufnahme des Steckers aufgenommen zu werden oder zu sein, wobei der Konnektor eine Mehrzahl von elektrischen Durchkontaktierungen aufweist, die entsprechend einer Anordnung der elektrischen Durchkontaktierungen des Steckers angeordnet sind, wobei die elektrischen Durchkontaktierungen des Konnektors proximal mit einem Flexboard verbunden sind, das eine planare Lötpad-Struktur mit Lötpads aufweist, an denen die Signal- und Verbindungskabel angelötet sind, wobei der Stecker und das Konnektorgehäuse an ihren Außenseiten Strukturen, insbesondere Außengewinde und Abschlussflächen, aufweisen, die mittels Aufschrauben einer Überwurfmutter ein Anpressen und Fixieren der Steckverbindung bewirken.

Erfindungsgemäß wird die Herstellung der Steckverbindung durch die konnektorseitige Verwendung eines Flexboards mit einer planaren Lötpad-Struktur zur Kontaktierung der zum Videoendoskop führenden Kabel vereinfacht. Flexboards waren bislang schon zur Kontaktierung in der Videoeinheit eingesetzt worden. Die Kontaktierung auf der Seite der Kabeleinheit ist jedoch mechanisch stark belastet, weshalb der Einsatz von Flexboards bislang nicht in Erwägung gezogen worden war.

Die erfindungsgemäße Steckverbindung ist wie bisher für die Videoleitungen des Videoendoskops einsetzbar, aber auch beispielsweise für Aktuatorleitungen und/oder Heater-Leitungen. Die Kontaktierung der Litzen und Schirme der Kabel auf den Lötpads des Flexboards ist wesentlich einfacher als bisher, und die Konfektionierung der Kabel wird ebenfalls erleichtert. Diese Ausführung führt außerdem zu einem wesentlich kleineren Sprung der Transferimpedanz als bisher.

Erfindungsgemäß weisen der Stecker und das Konnektorgehäuse an ihren Außenseiten Strukturen, insbesondere Außengewinde und Abschlussflächen, auf, die mittels Aufschrauben einer Überwurfmutter ein Anpressen und Fixieren der Steckverbindung bewirken. Diese Maßnahme führt zu einer kleinbauenden, aber sicheren Verbindung des Steckkontakts, die auch noch Platz für andere Teile eines Endoskops lässt, beispielsweise Lichtleitfasern oder Kanäle.

Das Konnektorgehäuse weist in Ausführungsformen im Bereich des Flexboards eine geringere Querschnittsfläche auf als im Bereich des Konnektors. Dies ist durch die Verwendung eines Flexboards mit einer planaren Lötpad-Struktur möglich geworden. Da sich das Konnektorgehäuse nach proximal hin verjüngt, kann das Instrument insgesamt schlanker und handlicher gebaut werden. Gleichzeitig ist im Handgriff mehr Platz für weitere Teile vorhanden, beispielsweise Bedienelemente oder Elektronik.

Die Herstellung der Verbindung wird vorteilhafterweise vereinfacht und sicher ausgestaltet, wenn die Konnektoraufnahme und das Konnektorgehäuse zueinander komplementäre Strukturen zur korrekten Ausrichtung des Konnektors und des Steckers zueinander aufweisen.

In Ausführungsformen ist das Konnektorgehäuse mittels einer in eine proximale Aufnahme des Konnektorgehäuses passende Ferrule mit Durchführungen für die Signal- und Versorgungskabel abgeschlossen und sind die Signal- und Versorgungskabel in die Ferrule eingelötet. Unter einer Ferrule wird ein Stopfen mit Durchgangsöffnungen für die Kabel verstanden. Die Ferrule kann ihrerseits in das Konnektorgehäuse eingelötet werden. Diese Maßnahmen sorgen für eine hermetische und mechanisch stabile Durchführung der Kabel in das Konnektorgehäuse.

Die Ferrule und die proximale Aufnahme des Konnektorgehäuses weisen in Ausführungsformen zueinander passende Formen auf, die eine Rotation der Ferrule in der proximalen Aufnahme des Konnektorgehäuses verhindern. Solche Formen sind vorzugsweise rotationsasymmetrisch. Im Querschnitt sind sie beispielsweise abgeplattet, eckig oder abgeflacht. Diese Maßnahme sorgt dafür, dass nur eine Orientierung oder aber zwei um 180° zueinander gedrehte Orientierungen angeboten wird bzw. werden, was den Zusammenbau vereinfacht. Zusätzlich können sich die Signal- und Versorgungskabel nicht durch eine unerwünschte Verdrehung der Ferrule in der Aufnahme des Konnektorgehäuses verdrillen und verkürzen.

Das Konnektorgehäuse ist in Ausführungsformen mit einem Verguss vollständig ausgefüllt. Der vollständige Verguss sichert die Verbindung zwischen den Kabeln und den Lötpads auf dem Flexboard mechanisch vollständig ab und reduziert schädliche mechanische Beanspruchungen der Lötstellen und der Kabel in optimaler Weise.

Zur Vereinfachung der Montage ist das Konnektorgehäuse in Ausführungsformen zweiteilig ausgebildet, wobei der Konnektor in ein distales Konnektorgehäuseteil eingepasst ist und die Ferrule in ein proximales Konnektorgehäuseteil eingepasst ist. Das proximale und das distale Konnektorgehäuseteil sind oder werden zu dem Konnektorgehäuse verbunden, beispielsweise durch Verpressen, insbesondere, bevor das Konnektorgehäuse durch eine Einfüllöffnung mit einem Verguss gefüllt wird. Die Zweiteiligkeit des Gehäuses erlaubt es, den Konnektor und die Ferrule getrennt voneinander in das jeweilige Konnektorgehäuseteil einzubringen, so dass die Handhabung stark vereinfacht wird.

Die der Erfindung zugrundeliegende Aufgabe wird auch durch ein Videoendoskop mit einer distalen Videoeinheit und einer proximalen Kabeleinheit sowie einer erfindungsgemäßen, zuvor beschriebenen, Steckverbindung zur Herstellung einer Signalverbindung zwischen der distalen Videoeinheit und der proximalen Kabeleinheit gelöst. Das Videoendoskop, das eine zuvor beschriebene Ausführungsform der erfindungsgemäßen Steckverbindung umfasst, verwirklicht damit alle zuvor beschriebenen Vorteile, Eigenschaften und Merkmale.

Die der Erfindung zugrundeliegende Aufgabe wird auch durch ein Verfahren zur Herstellung einer zuvor beschriebenen erfindungsgemäßen Steckverbindung zur Herstellung einer Signalverbindung zwischen einer distalen Videoeinheit und einer proximalen Kabeleinheit eines zuvor beschriebenen erfindungsgemäßen Videoendoskops, mit den folgenden Schritten gelöst:
- das Flexboard wird an die elektrischen Durchkontaktierungen des Konnektors auf einer proximalen Seite des Konnektors angelötet,
- Seitenflächen des Flexboards werden hochgebogen und der Konnektor wird in das Konnektorgehäuse eingepresst,
- die Signal- und Verbindungskabel werden an die Lötpads der planaren Lötpad-Struktur des Flexboards angelötet,
- die Signal- und Verbindungskabel werden vor oder nach dem Anlöten an die planare Lötpad-Struktur in die Ferrule eingelötet,
- das Konnektorgehäuse wird geschlossen, und
- die Ferrule wird mit dem Konnektorgehäuse verlötet.

Mit diesem Verfahren wird der zuvor beschriebene erfindungsgemäße Steckkontakt hergestellt. Es verwirklicht somit die gleichen Vorteile, Merkmale und Eigenschaften wie die anderen Erfindungsgegenstände.

In Ausführungsformen des Verfahrens wird das Konnektorgehäuse mit einem Verguss vollständig ausgefüllt.

Vorteilhafterweise werden am proximalen Ende der distalen Videoeinheit elektrische Durchkontaktierungen eines als Glasvergussteil ausgebildeten Steckers mit Kontaktflächen eines Flexboards verbunden, das mit Signal- und Versorgungskabeln in der distalen Videoeinheit verbunden ist, und der Stecker wird in ein Gehäuse der distalen Videoeinheit so eingesteckt und mit dem Gehäuse der distalen Videoeinheit verbunden, dass ein Teil des Steckers nach proximal aus dem Gehäuse herausragt. So können Außenkonturen des Steckers zur Sicherung verwendet werden, etwa durch ein Außengewinde für eine Überwurfmutter.

Zum Schließen des Konnektorgehäuses wird in Ausführungsformen ein proximales Konnektorgehäuseteil auf oder in ein distales Konnektorgehäuseteil geschoben und mit dem distalen Konnektorgehäuseteil verpresst. Diese zweiteilige Ausbildung des Konnektorgehäuses erlaubt eine einfache Installation der elektrischen Kontaktierungen zwischen Konnektor und Flexboard sowie zwischen Flexboard und Kabeln.

Vorzugsweise wird die Steckverbindung mit einer Überwurfmutter gesichert, so dass eine feste und kleinbauende Sicherung verwirklicht ist.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1a) - 1c): eine bekannte Steckverbindung für ein Videoendoskop und
- Fig. 2a) - 2c): eine erfindungsgemäße Steckverbindung.

In den Zeichnungen sind jeweils gleiche oder gleichartige Elemente und/oder Teile mit denselben Bezugsziffern versehen, so dass von einer erneuten Vorstellung jeweils abgesehen wird.

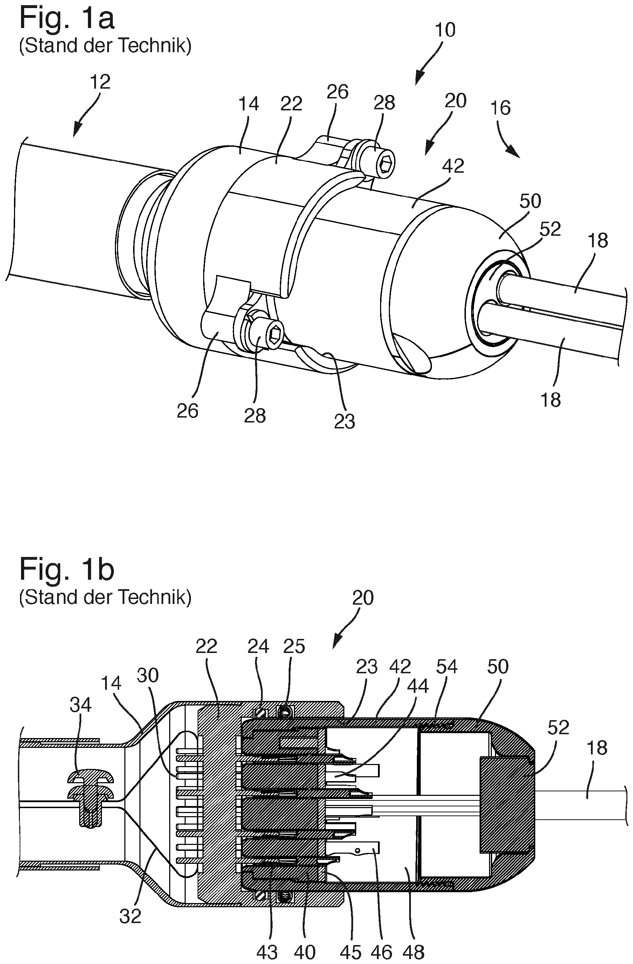

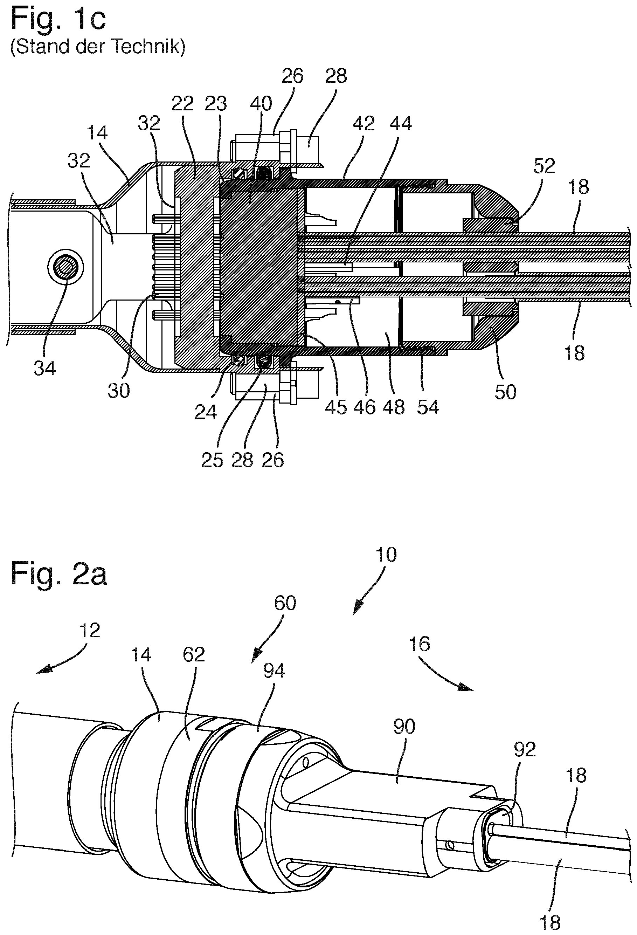

Die Fig. 1a) bis 1c) zeigen schematisch eine bekannte Steckverbindung 20 eines Videoendoskops 10. Die Steckverbindung 20 ist in Figur 1a) verkleidet, während sie in den Figuren 1b) und 1c) in zwei senkrechten Schnitten dargestellt ist. Der Schnitt in Figur 1b) verläuft senkrecht zwischen den beiden Kabeln hindurch, während die Schnittebene in Figur 1c) senkrecht dazu in einer Ebene verläuft, die durch die beiden Schrauben 28 verläuft, also oberhalb der zentralen Achse der im wesentlichen zylindrischen Steckverbindung 20.

In Figur 1a) ist dargestellt, dass die Steckverbindung 20 zu einer Seite an eine distale Videoeinheit 12 angrenzt und zur gegenüberliegenden Seite an die proximale Kabeleinheit 16. Die distale Videoeinheit 12 ist üblicherweise in einem Endoskopschaft des Videoendoskops 10 angeordnet, während der proximale Teil in einem Handgriff des Videoendoskops 10 angeordnet ist. Auf der distalen Seite ist die Steckverbindung 20 in ein Gehäuse 14 eingefasst, auf der proximalen Seite in ein Konnektorgehäuse 42, welches mit einem Deckel 50 nach proximal abgeschlossen ist. Die Kabel 18 führen durch eine Ferrule 52 mit Öffnungen für die Kabel 18 in das Konnektorgehäuse 42 der Steckverbindung 20 hinein. Zur Abdichtung ist die Ferrule 52 in den Deckel 50 eingelötet und sind die Kabel 18 in die Ferrule 52 eingelötet. Auf der Seite der distalen Videoeinheit 12 ist ein Stecker 22 in einen erweiterten Teil des Gehäuses 14 eingepresst und ragt nach proximal aus diesem Gehäuse 14 heraus. In diesem Bereich ist der Stecker 22 innen hohl und weist eine Konnektoraufnahme 23 auf, in die der Konnektor 40 mit seinem Konnektorgehäuse 42 eingeführt ist. Die Steckverbindung wird durch eine Verschraubung an zwei Augen 26 mit Innengewindebohrung und zwei Schrauben 28 mit Federscheiben gesichert, wobei auch das Konnektorgehäuse 42 entsprechende Augen (ohne Bezugszeichen) aufweist, die mit den Augen 26 des Steckers 22 fluchten, bei dem es sich beispielsweise um einen 30-Pin-Stecker handeln kann.

Bei den Kabeln 18 kann es sich um Signalkabel für die Videosignale, aber auch um Versorgungskabel zur Versorgung der Videoeinheit 12 mit Strom oder von anderen Instrumenten handeln.

In Figur 1b) ist ein Querschnitt der Steckverbindung 20 gezeigt, die senkrecht zwischen den beiden Kabeln 18 hindurch verläuft. Zentral ist auf der distalen Seite der massive Stecker 22, der als Glasverguss ausgeführt sein kann, dargestellt. Dieser hat in dem gezeigten Querschnitt eine U-Form, deren Basis nach distal zeigt und von elektrischen Durchkontaktierungen 30 (Pins) durchsetzt ist, die hermetisch in den Glasverguss eingelassen sind. Die elektrischen Durchkontaktierungen 30 ragen nach distal aus dem Stecker 22 heraus und sind mit einer kreisrunden Basis eines Flexboards 32 verbunden, das Leiterbahnen aufweist sowie Lötpads (nicht gezeigt), an denen die Durchkontaktierungen 30 elektrisch mit den Lötpads bzw. Leiterbahnen des Flexboards 32 verbunden sind. Zwei Arme des Flexboards 32 sind zueinander hingebogen und werden mittels eines nichtleitenden Verbindungselements 34 in der Form beieinander gehalten. Das nicht gezeigte distale Ende des Flexboards 32 kann beispielsweise in eine entsprechende Buchse (nicht gezeigt) eingesteckt sein.

Der Stecker 22 hat nach proximal weisend einen im wesentlichen zylindrischen Rand, der in dem gezeigten Querschnitt die Arme der U-Form darstellen, und dessen Innenseite eine Aufnahme 23 für den Konnektor 40 der Steckverbindung 20 bildet, der die Verbindung mit den Kabeln 18 nach proximal bildet. Dieser Konnektor 40 weist ebenfalls eine gleich große Anzahl von elektrischen Durchkontaktierungen (44) auf (Pins), die auf der distalen Seite als Hohlpins in Aufnahmen 43 für die Durchkontaktierungen 30 ausgebildet sind und somit einen sicheren elektrischen Kontakt mit den elektrischen Durchkontaktierungen 30 des Steckers 22 herstellen, wenn der Konnektor 40 in den Stecker 22 eingesteckt wird.

Die Herstellung des Konnektors 40 ist aufwendig. Zunächst erfolgt die Kontaktierung der einzelnen Signalleiter der Kabel 18 mit dem Konnektor 40. Dies erfolgt durch Anlöten der Litzen und der Schirmgewebe der Signalleitungen aus dem Kabel 18 an die nach proximal herausschauenden Enden der elektrischen Durchkontaktierungen 44, gegebenenfalls unterstützt durch Lötadapter 46, auch Lötkelche genannt. Dazu werden die Kabel 18 manuell in einem mehrstufigen Prozess abisoliert und der äußere Schirm in die Ferrule 52 eingelötet. Danach werden die einzelnen Litzen in die Lötkelche des Konnektors 40 eingelötet, teilweise wird dazu noch zusätzlich ein Lötadapter für die dickeren Litzen benötigt. Viele der Einzelleitungen sind Koaxialkabel. Deren Schirme werden verdrillt und in Gruppen zusammengefasst und auch in Lötkelche gelötet.

Anschließend wird der Konnektor 40 in ein wiederum im Wesentlichen zylindrisches Konnektorgehäuse 42 eingepresst und der Innenraum mit einem Verguss aus einem Dickschichtlack bis zum Gewinde einer Schraubverbindung 54 vergossen, um die Kontaktierungen mechanisch und chemisch zu schützen. Das Innengewinde für die Schraubverbindung 54 bleibt vom Verguss 48 ausgenommen. Ferner ist die proximale Seite des Konnektors 40 durch eine Abdeckscheibe 45 geschützt, durch die die elektrischen Durchkontaktierungen 44 ebenfalls hindurchragen. Die Abdeckscheibe 45 wird durch formkomplementäre Strukturen in der Abdeckscheibe 45 und dem Konnektor 40 in Position gebracht und gehalten. Nach dem Aushärten des Vergusses 48 wird ein Deckel 50 von proximal in die Schraubverbindung 54 des Konnektorgehäuses 42 eingeschraubt und die Ferrule 52, durch die die Kabel 18 geführt sind, in die Auslassöffnung des Deckels 50 gezogen und dort verlötet.

Der so fertiggestellte Konnektor 40 wird in den Stecker 22 eingesteckt und die Steckverbindung dann mit zwei Schrauben 28 mit Federscheiben gesichert, die durch Augen (ohne Bezugszeichen) des Konnektorgehäuses 42 geführt und in Augen 26 des Steckers 22 mit Innengewindebohrung geschraubt werden.

Die Außenkonturen des Konnektorgehäuses 42 stimmen mit der Innenkontur des Randes des Steckers 22 überein, und die vorzugsweise komplementären Formen enthalten vorzugsweise komplementäre Strukturen, die eine korrekte Ausrichtung des Konnektors 40 in Bezug auf den Stecker 22 und die jeweiligen elektrischen Durchkontaktierungen 30, 44 beim Einstecken gewährleisten. Ein sicherer Sitz sowie eine hermetische Abdichtung werden durch einen umlaufenden O-Ring 24 und einen Spiralfederring 25 gewährleistet, die in umlaufenden Ausnehmungen im Rand des Steckers 22 angeordnet sind und auf das Konnektorgehäuse 42 drücken. Die Feder 25 und der O-Ring 24 bilden eine Schwachstelle, da sie nicht immer funktionieren und relativ schnell beschädigt werden können.

Wie in Figur 1c) dargestellt ist, wird die Steckverbindung 20, die durch Einstecken des Konnektors 40 in den Stecker 22 hergestellt wird, mittels Schrauben 28 gesichert, die durch jeweils ein Auge des Konnektorgehäuses 42 und durch ein Auge 26 des Steckers 22 geschraubt werden, wobei das Auge 26 des Steckers ein passendes Innengewinde für die Schraube 28 aufweist. Die Schraubverbindung mit den Schrauben 28 wird durch Federscheiben stabilisiert, die eine Vorspannung in die Schraubverbindung einbringen.

In den Figuren 2a) bis 2c) ist schematisch eine erfindungsgemäße Steckverbindung 60 gezeigt. Diese ist, wie in Figur 2a) deutlich zu erkennen, weniger ausladend als die bekannte Steckverbindung 20 aus Figur 1. Wiederum ist auf der linken Seite zu sehen, dass dieser Teil zur distalen Videoeinheit 12 gehört, während auf der rechten Seite die proximale Kabeleinheit 16 vorgesehen ist. Ebenfalls sind ein distales Gehäuse 14 mit dem Steckerteil und ein proximales Konnektorgehäuse 90 vorhanden, das allerdings deutlich schmaler ausgeführt ist als das Konnektorgehäuse 42 der bekannten Steckverbindung 20 aus Figur 1. Anstelle einer Schraubverbindung wird die Steckverbindung 60 mittels einer Überwurfmutter 94 gesichert, die über einen ausladenden Teil des Konnektorgehäuses 90 geworfen wird und auf ein Außengewinde des als Glasverguss ausgeführten Steckers 62 aufgeschraubt ist. Ein weiterer Unterschied besteht darin, dass die Ferrule 92 nicht notwendigerweise zylindrisch ist, sondern entsprechend der abgeplatteten Form des proximalen Endes des Gehäuses 90 ebenfalls abgeplattet sein kann, so dass sich die Kabel 18 nicht gegenüber dem Konnektorgehäuse 90 und den darin angeordneten Komponenten verdrehen können.

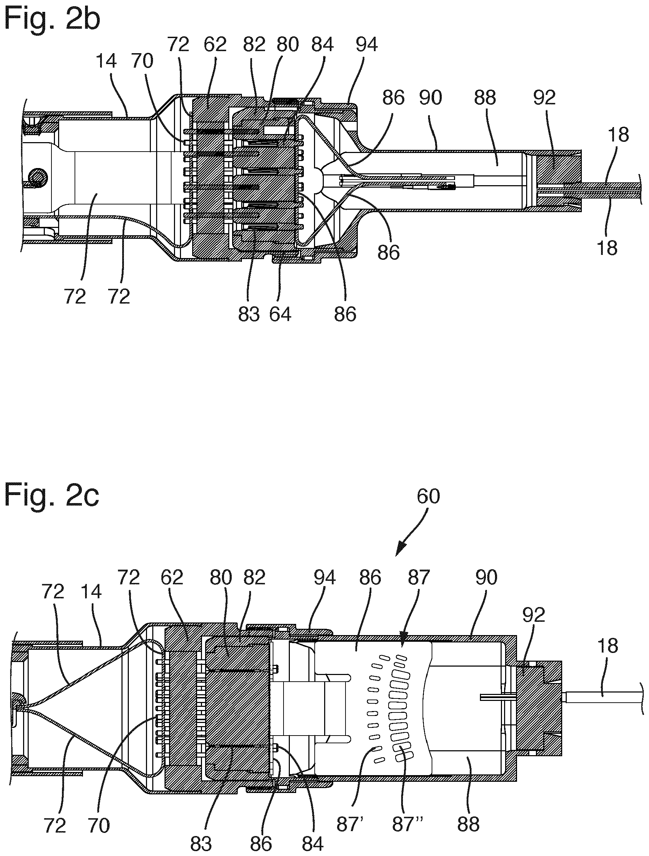

In den Figuren 2b) und 2c) sind zueinander senkrechte Schnitte durch die Steckverbindung 60 gezeigt, wobei der Schnitt in Figur 2b) senkrecht durch die zentrale Achse und der Schnitt in Figur 2c) waagerecht durch die zentrale Achse der Steckverbindung 60 verläuft.

Auf der distalen Seite ist der Stecker 62 mit der Verbindung zum Flexboard 72 funktionell ähnlich ausgestaltet wie in dem bekannten Beispiel aus der Figur 1. Der Stecker 62 ist allerdings in radialer Richtung nicht so ausladend wie der Stecker 22 aus Figur 1. Der Konnektor 80 ist in seinem zentralen Teil ebenfalls ähnlich dem Konnektor 40 aus Figur 1 ausgestaltet. Er wird allerdings nicht durch eine Abdeckscheibe 45 abgedeckt. Stattdessen ist bei dem erfindungsgemä-βen Konnektor in ähnlicher Weise wie bei dem Stecker 62 eine Verbindung zu einem Flexboard 86 realisiert, welches ebenfalls eine runde Basis aufweist mit den entsprechenden Durchgängen und Lötpads für die elektrischen Durchkontaktierungen 84 sowie zwei Armen, die sich aufbiegen und mit ihren Rückseiten zusammenlegen lassen, wie dies in Figur 2b) dargestellt ist. Die Durchkontaktierungen 84 sind nach distal wiederum in Aufnahmen 83 für die Durchkontaktierungen 70 des Steckers 62 angeordnet.

Der Stecker 62 hat ebenfalls eine Konnektoraufnahme 64 und elektrische Durchkontaktierungen 70 und weist am proximalen Ende seines Randes der Außenseite eine Gewindestruktur auf, die mit einer Innengewindestruktur der Überwurfmutter 94 zusammenarbeitet. Das Konnektorgehäuse 82 weist eine entsprechende umlaufende Erhebung auf, die von der Überwurfmutter 94 mitgenommen und an das proximale Ende des Steckers 62 gepresst wird, wodurch ein mechanisch sicherer und stabiler sowie hermetischer Anschluss der Steckverbindung 60 hergestellt wird.

Die Herstellung des erfindungsgemäßen Konnektors erfolgt dadurch, dass das Flexboard 86 an die elektrischen Durchkontaktierungen 84 des Konnektors 80 angelötet wird. Das Flexboard 86 kann dabei so gestaltet sein, dass es für mehrere Geräte verwendbar ist. Anschließend werden die Seitenflächen des Flexboards 86 hochgebogen und der Konnektor 80 in das distale Konnektorgehäuse 82 eingepresst. Das runde Muster der elektrischen Durchkontaktierungen 84 wird durch das Flexboard 86 in eine planare Lötpad-Struktur 87 umgesetzt, die Lötpads 87` für Signalleitungen und Lötpads 87" für die Ummantelung bzw. Abschirmung der Kabel aufweist. Die Schirme der Koaxialkabel werden nicht mehr miteinander verdrillt, sondern ebenso wie die Innenleiter auf eine einheitliche Länge abisoliert und gekürzt. Danach werden die Kabel 18 in die Ferrule 92 eingelötet. Die Kabel werden anschließend nebeneinander auf die gleiche Art und Weise auf die planare Lötpad-Struktur 87 des Flexboards 86 aufgelötet. Diese Ausführung erzeugt einen deutlich kleineren Sprung in der Transferimpedanz als die aus dem Stand der Technik gemäß Figur 1 bekannte Lösung.

Anschließend wird ein Deckel bzw. proximales Konnektorgehäuseteil 90, der ein- oder auch zweiteilig ausgeführt sein kann, von der Seite der Ferrule 92 übergeschoben und mit dem distalen Konnektorgehäuse 82 verpresst. In diesem proximalen Konnektorgehäuse 90, der nicht rund gestaltet ist, sind zwei Nuten integriert, die die Flächen des Flexboards 86 mittig in dem Konnektor positionieren. Auf diese Weise wird kein zusätzlicher Clip benötigt, um die Lötflächen mittig zu halten, und die Flächen des Flexboards 86 können komplett für das Routing der Leiterbahnen genutzt werden. Anschließend wird die Ferrule 92 mit dem Deckel bzw. proximalen Konnektorgehäuse 90 verlötet. Da die Konturen nicht mehr rund sind, sondern eckig, können sich die Kabellängen bei der Montage durch Rotation nicht mehr verkürzen. Dies wird dadurch unterstützt, dass die Ferrule 92 nicht zylindrisch geformt sein muss. Zum Abschluss wird der Konnektor durch eine Bohrung hindurch vergossen, bis die Vergussmasse des Vergusses 88 in Entlüftungsbohrungen zu sehen ist. Dies hat den Vorteil, dass der Konnektor vollständig vergossen ist und somit sämtliche mechanischen Belastungen von der Vergussmasse aufgenommen werden und die Lötkontaktierungen von mechanischen Belastungen freigehalten werden. Die Steckverbindung 60 wird dann mit der Überwurfmutter 94 gesichert. Diese Sicherung baut sehr viel weniger auf als bei dem bekannten Stecker, so dass die Verbindung in einen kleineren Handgriff passt und noch Platz beispielsweise für einen Lichtleitstecker bleibt.

### Bezugszeichenliste

- 10: Videoendoskop
- 12: distale Videoeinheit
- 14: Gehäuse
- 16: proximale Kabeleinheit
- 18: Signal- und Versorgungskabel
- 20: Steckverbindung
- 22: Stecker mit Glasverguss
- 23: Konnektoraufnahme
- 24: O-Ring
- 25: Spiralfederring
- 26: Auge mit Innengewindebohrung
- 28: Schraube mit Federscheibe
- 30: elektrische Durchkontaktierung (Pin)
- 32: Flexboard
- 34: Verbindungselement
- 40: Konnektor
- 42: Konnektorgehäuse
- 43: Aufnahme
- 44: elektrische Durchkontaktierung (Pin)
- 45: Abdeckscheibe
- 46: Lötadapter
- 48: Verguss
- 50: Deckel
- 52: Ferrule
- 54: Schraubverbindung
- 60: Steckverbindung
- 62: Stecker mit Glasverguss
- 64: Konnektoraufnahme
- 70: elektrische Durchkontaktierung (Pin)
- 72: Flexboard
- 80: Konnektor
- 82: distales Konnektorgehäuseteil
- 83: Aufnahme
- 84: elektrische Durchkontaktierung (Pin)
- 86: Flexboard
- 87: Lötpad-Struktur
- 87`: Lötpad für Signalleitung
- 87": Lötpad für Ummantelung (Masse)
- 88: Verguss
- 90: proximales Konnektorgehäuseteil
- 92: Ferrule
- 94: Überwurfmutter

## Patentansprüche

1. Steckverbindung (60) für ein Videoendoskop (10) zur Herstellung einer Signalverbindung zwischen einer distalen Videoeinheit (12) und einer proximalen Kabeleinheit (16) des Videoendoskops (10), umfassend einen Stecker (62) und einen komplementären Konnektor (80), der zur Herstellung und Unterbrechung der Signalverbindung in den Stecker (62) einsteckbar ist, wobei der Stecker (62) als Glasvergussteil geformt und an einem proximalen Ende der distalen Videoeinheit (10) angeordnet ist und der Konnektor (80) an einem distalen Ende der proximalen Kabeleinheit (16) angeordnet ist, wobei der Stecker (62) an seiner Außenseite mit einem Gehäuse (14) der distalen Videoeinheit (12) verbunden ist, eine Mehrzahl von elektrischen Durchkontaktierungen (70) aufweist, auf einer distalen Seite mit einem Flexboard (72) in der distalen Videoeinheit (12) elektrisch leitend verbunden ist und auf seiner proximalen Seite eine als Aussparung ausgebildete Konnektoraufnahme (64) für den Konnektor (80) aufweist, wobei der Konnektor (80) in einem Konnektorgehäuse (82, 90) eingefasst ist, welches an seiner Außenseite passend zu der Konnektoraufnahme (64) des Steckers (62) geformt ist, um formschlüssig und/oder kraftschlüssig in der Konnektoraufnahme (64) des Steckers (62) aufgenommen zu werden oder zu sein, wobei der Konnektor (80) eine Mehrzahl von elektrischen Durchkontaktierungen (84) aufweist, die entsprechend einer Anordnung der elektrischen Durchkontaktierungen (70) des Steckers (62) angeordnet sind, wobei die elektrischen Durchkontaktierungen (84) des Konnektors (80) proximal mit einem Flexboard (86) verbunden sind, das eine planare Lötpad-Struktur mit Lötpads aufweist, an denen die Signal- und Verbindungskabel (18) angelötet sind, **dadurch gekennzeichnet, dass** der Stecker (62) und das Konnektorgehäuse (82, 90) an ihren Außenseiten Strukturen, insbesondere Außengewinde und Abschlussflächen, aufweisen, die mittels Aufschrauben einer Überwurfmutter (94) ein Anpressen und Fixieren der Steckverbindung (60) bewirken.

2. Steckverbindung (60) nach Anspruch 1, **dadurch gekennzeichnet, dass** das Konnektorgehäuse (82, 90) im Bereich des Flexboards (86) eine geringere Querschnittsfläche aufweist als im Bereich des Konnektors (80).

3. Steckverbindung (60) nach Anspruch 1 oder 2, **dadurch gekennzeichnet, dass** die Konnektoraufnahme (64) und das Konnektorgehäuse (82, 90) zueinander komplementäre Strukturen zur korrekten Ausrichtung des Konnektors (80) und des Steckers (62) zueinander aufweisen.

4. Steckverbindung (60) nach einem der Ansprüche 1 bis 3, **dadurch gekennzeichnet, dass** das Konnektorgehäuse (82, 90) mittels einer in eine proximale Aufnahme des Konnektorgehäuses (82, 90) passende Ferrule (92) mit Durchführungen für die Signal- und Versorgungskabel (18) abgeschlossen ist und die Signal- und Versorgungskabel (18) in die Ferrule (92) eingelötet sind.

5. Steckverbindung nach Anspruch 4, **dadurch gekennzeichnet, dass** die Ferrule (92) und die proximale Aufnahme des Konnektorgehäuses (82, 90) zueinander passende Formen aufweisen, die eine Rotation der Ferrule (92) in der proximalen Aufnahme des Konnektorgehäuses verhindern.

6. Steckverbindung nach Anspruch 4 oder 5, **dadurch gekennzeichnet, dass** das Konnektorgehäuse (82, 90) mit einem Verguss (88) vollständig ausgefüllt ist.

7. Steckverbindung nach einem der Ansprüche 4 bis 6, **dadurch gekennzeichnet, dass** das Konnektorgehäuse (82, 90) zweiteilig ausgebildet ist, wobei der Konnektor (80) in ein distales Konnektorgehäuseteil (82) eingepasst ist und die Ferrule (92) in ein proximales Konnektorgehäuseteil eingepasst ist.

8. Videoendoskop mit einer distalen Videoeinheit und einer proximalen Kabeleinheit sowie einer Steckverbindung nach einem der Ansprüche 1 bis 7 zur Herstellung einer Signalverbindung zwischen der distalen Videoeinheit und der proximalen Kabeleinheit.

9. Verfahren zur Herstellung einer Steckverbindung (60) nach einem der Ansprüche 4 bis 7 für ein Videoendoskop (10) zur Herstellung einer Signalverbindung zwischen einer distalen Videoeinheit (12) und einer proximalen Kabeleinheit (16) des Videoendoskops (10), mit den folgenden Schritten:
- das Flexboard (86) wird an die elektrischen Durchkontaktierungen (84) des Konnektors (80) auf einer proximalen Seite des Konnektors (80) angelötet,
- Seitenflächen des Flexboards (86) werden hochgebogen und der Konnektor (80) wird in das Konnektorgehäuse (82) eingepresst,
- die Signal- und Verbindungskabel (18) werden an die Lötpads (87', 87") der planaren Lötpad-Struktur (87) des Flexboards (86) angelötet,
- die Signal- und Verbindungskabel (18) werden vor oder nach dem Anlöten an die planare Lötpad-Struktur (87) in die Ferrule (92) eingelötet,
- das Konnektorgehäuse (82, 90) wird geschlossen, und
- die Ferrule (92) wird mit dem Konnektorgehäuse (82, 90) verlötet.

10. Verfahren nach Anspruch 9, **dadurch gekennzeichnet, dass** das Konnektorgehäuse (82, 90) mit einem Verguss (88) vollständig ausgefüllt wird.

11. Verfahren nach Anspruch 9 oder 10, **dadurch gekennzeichnet, dass** am proximalen Ende der distalen Videoeinheit (12) elektrische Durchkontaktierungen (70) eines als Glasvergussteil ausgebildeten Steckers (62) mit Kontaktflächen eines Flexboards (72) verbunden werden, das mit Signal- und Versorgungskabeln in der distalen Videoeinheit (12) verbunden ist, und der Stecker (62) in ein Gehäuse (14) der distalen Videoeinheit (12) so eingesteckt und mit dem Gehäuse (14) der distalen Videoeinheit (12) verbunden wird, dass ein Teil des Steckers (62) nach proximal aus dem Gehäuse (14) herausragt.

12. Verfahren nach einem der Ansprüche 9 bis 11, **dadurch gekennzeichnet, dass** zum Schließen des Konnektorgehäuses (82, 90) ein proximales Konnektorgehäuseteil (90) auf oder in ein distales Konnektorgehäuseteil (82) geschoben und mit dem distalen Konnektorgehäuseteil (82) verpresst wird.

13. Verfahren nach einem der Ansprüche 9 bis 12, **dadurch gekennzeichnet, dass** die Steckverbindung mit einer Überwurfmutter gesichert wird.

## Claims

1. A plug connection (60) for a video endoscope (10) for producing a signal connection between a distal video unit (12) and a proximal cable unit (16) of the video endoscope (10), comprising a plug (62) and a complementary connector (80) which can be inserted into the plug (62) in order to produce and interrupt the signal connection, wherein the plug (62) is formed as a glass cast part and is arranged at a proximal end of the distal video unit (10) and the connector (80) is arranged at a distal end of the proximal cable unit (16), wherein the plug (62) is connected on its outer side to a housing (14) of the distal video unit (12), has a plurality of electrical feedthroughs (70), is connected in an electrically conducting manner on a distal side to a flexboard (72) in the distal video unit (12) and has on its proximal side a connector receptacle (64) configured as a recess for the connector (80), wherein the connector (80) is enclosed in a connector housing (82, 90) which is formed on its outer side to fit the connector receptacle (64) of the plug (62) in order to become or be received in the connector receptacle (64) of the plug (62) by form-fit and/or by force-fit, wherein the connector (80) has a plurality of electrical feedthroughs (84) which are arranged in accordance with an arrangement of the electrical feedthroughs (70) of the plug (62), wherein the electrical feedthroughs (84) of the connector (80) are connected proximally to a flexboard (86) which has a planar solder pad structure having solder pads, to which the signal and connecting cables (18) are soldered, **characterized in that** the plug (62) and the connector housing (82, 90) have structures on their outer sides, in particular external threads and termination surfaces, which cause the plug connection (60) to be pressed on and fixed by means of screwing on a union nut (94).

2. The plug connection (60) according to Claim 1, **characterized in that** the connector housing (82, 90) has a smaller cross-sectional area in the region of the flexboard (86) than in the region of the connector (80).

3. The plug connection (60) according to Claim 1 or 2, **characterized in that** the connector receptacle (64) and the connector housing (82, 90) have mutually complementary structures in order to correctly align the connector (80) and the plug (62) with respect to one another.

4. The plug connection (60) according to any one of Claims 1 to 3, **characterized in that** the connector housing (82, 90) is terminated by means of a ferrule (92) which fits into a proximal receptacle of the connector housing (82, 90), said ferrule having leadthroughs for the signal and supply cables (18), and the signal and supply cables (18) are soldered into the ferrule (92).

5. The plug connection according to Claim 4, **characterized in that** the ferrule (92) and the proximal receptacle of the connector housing (82, 90) have shapes which match one another, which prevent a rotation of the ferrule (92) in the proximal receptacle of the connector housing.

6. The plug connection according to Claim 4 or 5, **characterized in that** the connector housing (82, 90) is completely filled up with a casting (88).

7. The plug connection according to any one of Claims 4 to 6, **characterized in that** the connector housing (82, 90) is configured in two parts, wherein the connector (80) is fitted into a distal connector housing part (82) and the ferrule (92) is fitted into a proximal connector housing part.

8. A video endoscope having a distal video unit and a proximal cable unit as well as a plug connection according to any one of Claims 1 to 7 for producing a signal connection between the distal video unit and the proximal cable unit.

9. A method for producing a plug connection (60) according to any one of Claims 4 to 7 for a video endoscope (10) for producing a signal connection between a distal video unit (12) and a proximal cable unit (16) of the video endoscope (10) according to Claim 8, having the following steps:
- the flexboard (86) is soldered to the electrical feedthroughs (84) of the connector (80) on a proximal side of the connector (80),
- side surfaces of the flexboard (86) are bent upwards and the connector (80) is pressed into the connector housing (82),
- the signal and connecting cables (18) are soldered to the solder pads (87', 87") of the planar solder pad structure (87) of the flexboard (86),
- the signal and connecting cables (18) are soldered into the ferrule (92) before or after soldering to the planar solder pad structure (87),
- the connector housing (82, 90) is closed, and
- the ferrule (92) is soldered to the connector housing (82, 90).

10. The method according to Claim 9, **characterized in that** the connector housing (82, 90) is completely filled up with a casting (88).

11. The method according to Claim 9 or 10, **characterized in that** at the proximal end of the distal video unit (12), electrical feedthroughs (70) of a plug (62) configured as a glass cast part are connected to contact surfaces of a flexboard (72), which is connected to signal and supply cables in the distal video unit (12), and the plug (62) is inserted into a housing (14) of the distal video unit (12) and is connected to the housing (14) of the distal video unit (12) such that a part of the plug (62) projects proximally out of the housing (14).

12. The method according to any one of Claims 9 to 11, **characterized in that**, in order to close the connector housing (82, 90), a proximal connector housing part (90) is pushed onto or into a distal connector housing part (82) and is pressed with the distal connector housing part (82).

13. The method according to any one of Claims 9 to 12, **characterized in that** the plug connection is secured with a union nut.

## Revendications

1. Liaison enfichable (60) pour un vidéo-endoscope (10) destinée à établir une connexion de signal entre une unité vidéo distale (12) et une unité de câble proximale (16) du vidéo-endoscope (10), comprenant une fiche (62) et un connecteur complémentaire (80), qui est apte à être enfiché dans la fiche (62) pour établir et interrompre la connexion de signal, la fiche (62) étant formée en tant que pièce moulée en verre et étant agencée à une extrémité proximale de l'unité vidéo distale (10) et le connecteur (80) étant agencé à une extrémité distale de l'unité de câble proximale (16), la fiche (62) étant reliée sur son côté extérieur à un boîtier (14) de l'unité vidéo distale (12), présentant une pluralité de connexions électriques traversantes (70), étant reliée de manière électriquement conductrice sur un côté distal à une carte flexible (72) située dans l'unité vidéo distale (12) et présentant sur son côté proximal un logement de connecteur (64) conçu sous la forme d'un évidement pour le connecteur (80), le connecteur (80) étant enchâssé dans un boîtier de connecteur (82, 90) qui est formé sur son côté extérieur de manière à s'adapter au logement de connecteur (64) de la fiche (62), afin d'être reçu ou d'être apte à être reçu par complémentarité de forme et/ou par force dans le logement de connecteur (64) de la fiche (62), le connecteur (80) présentant une pluralité de connexions électriques traversantes (84) qui sont agencées en fonction d'une disposition des connexions électriques traversantes (70) de la fiche (62), les connexions électriques traversantes (84) du connecteur (80) étant reliées de manière proximale à une carte flexible (86) qui présente une structure plane de plots de soudage avec des plots de soudage sur lesquels sont soudés les câbles de signal et de connexion (18), **caractérisée en ce que** la fiche (62) et le boîtier du connecteur (82, 90) présentent sur leurs côtés extérieurs des structures, en particulier des filetages extérieurs et des surfaces de fermeture, qui font que la liaison enfichable (60) est pressée et fixée par vissage d'un écrou-raccord (94).

2. Liaison enfichable (60) selon la revendication 1, **caractérisée en ce que** le boîtier de connecteur (82, 90) présente une surface de section transversale plus petite dans la zone de la carte flexible (86) que dans la zone du connecteur (80).

3. Liaison enfichable (60) selon la revendication 1 ou la revendication 2, **caractérisée en ce que** le logement de connecteur (64) et le boîtier de connecteur (82, 90) présentent des structures complémentaires l'une à l'autre pour l'orientation correcte du connecteur (80) et de la fiche (62) l'un par rapport à l'autre.

4. Liaison enfichable (60) selon l'une des revendications 1 à 3, **caractérisée en ce que** le boîtier de connecteur (82, 90) est fermé au moyen d'une ferrule (92) placée dans un logement proximal du boîtier de connecteur (82, 90) et comportant des passages pour les câbles de signal et d'alimentation (18), et **en ce que** les câbles de signal et d'alimentation (18) sont soudés dans la ferrule (92).

5. Liaison enfichable selon la revendication 4, **caractérisée en ce que** la ferrule (92) et le logement proximal du boîtier de connecteur (82, 90) présentent des formes adaptées l'une à l'autre, qui empêchent une rotation de la ferrule (92) dans le logement proximal du boîtier de connecteur.

6. Liaison enfichable selon la revendication 4 ou la revendication 5, **caractérisée en ce que** le boîtier de connecteur (82, 90) est entièrement rempli d'un scellement (88).

7. Liaison enfichable selon l'une des revendications 4 à 6, **caractérisée en ce que** le boîtier de connecteur (82, 90) est formé en deux parties, le connecteur (80) étant ajusté dans une partie distale du boîtier de connecteur (82) et la férule (92) étant ajustée dans une partie proximale du boîtier de connecteur.

8. Vidéo-endoscope comprenant une unité vidéo distale et une unité de câble proximale ainsi qu'une liaison enfichable selon l'une des revendications 1 à 7 pour établir une connexion de signal entre l'unité vidéo distale et l'unité de câble proximale.

9. Procédé de fabrication d'une liaison enfichable (60) selon l'une des revendications 4 à 7 pour un vidéo-endoscope (10) destinée à établir une connexion de signal entre une unité vidéo distale (12) et une unité de câble proximale (16) du vidéo-endoscope (10), comprenant les étapes suivantes :
- la carte flexible (86) est soudée aux connexions électriques traversantes (84) du connecteur (80) sur un côté proximal du connecteur (80),
- les surfaces latérales de la carte flexible (86) sont pliées vers le haut et le connecteur (80) est pressé dans le boîtier du connecteur (82),
- les câbles de signal et de connexion (18) sont soudés aux plots de soudure (87', 87") de la structure plane des plots de soudure (87) de la carte flexible (86),
- les câbles de signal et de connexion (18) sont soudés dans la ferrule (92) avant ou après la soudure à la structure plane des plots de soudure (87),
- le boîtier du connecteur (82, 90) est fermé, et
- la ferrule (92) est soudée au boîtier de connecteur (82, 90).

10. Procédé selon la revendication 9, **caractérisé en ce que** le boîtier de connecteur (82, 90) est entièrement rempli d'un moulage (88).

11. Procédé selon la revendication 9 ou la revendication 10, **caractérisé en ce qu'**à l'extrémité proximale de l'unité vidéo distale (12), des connexions électriques traversantes (70) d'une fiche (62) réalisée sous forme de pièce moulée en verre sont reliées à des surfaces de contact d'une carte flexible (72), qui est reliée à des câbles de signal et d'alimentation dans l'unité vidéo distale (12), et la fiche (62) est enfichée dans un boîtier (14) de l'unité vidéo distale (12) et est connecté au boîtier (14) de l'unité vidéo distale (12) de telle sorte qu'une partie de la fiche (62) dépasse du boîtier (14) vers le côté proximal.

12. Procédé selon l'une des revendications 9 à 11, **caractérisé en ce que**, pour fermer le boîtier de connecteur (82, 90), une partie proximale du boîtier de connecteur (90) est glissée sur ou dans une partie distale du boîtier de connecteur (82) et est comprimée avec la partie distale du boîtier de connecteur (82).

13. Procédé selon l'une des revendications 9 à 12, **caractérisé en ce que** la liaison enfichable est sécurisée par un écrou-raccord.
